(19) Europäisches Patentamt
European Patent Office
Office européen des brevets

(11) Publication number: **0 206 362**
**B1**

(12) # EUROPEAN PATENT SPECIFICATION

(45) Date of publication of patent specification: **17.10.90**

(51) Int. Cl.$^5$: **A 61 K 6/08**, C 09 J 133/10, C 08 L 51/00

(21) Application number: **86200332.4**

(22) Date of filing: **15.08.83**

(60) Publication number of the earlier application in accordance with Art. 76 EPC: **0 103 420**

(54) **Adhesive coating material.**

(30) Priority: **16.08.82 JP 141010/82**
**24.11.82 JP 204683/82**

(43) Date of publication of application:
**30.12.86 Bulletin 86/52**

(45) Publication of the grant of the patent:
**17.10.90 Bulletin 90/42**

(84) Designated Contracting States:
**DE FR GB NL SE**

(56) References cited:
**EP-A-0 017 937**
**DE-A-2 739 282**
**FR-A-2 505 347**
**GB-A-1 495 255**
**US-A-4 005 247**

(73) Proprietor: **TOKUYAMA SODA KABUSHIKI KAISHA**
**1-1 Mikage-cho**
**Tokuyama-shi Yamaguchi-ken (JP)**

(72) Inventor: **Kusumoto, Koshi**
**811-32 Kajihara**
**Kamakura-shi Kanagawa-ken (JP)**
Inventor: **Ogata, Takayuki**
**9-901 Dream Height 1403, Matano-cho Totsuka-ku**
**Yokohama (JP)**
Inventor: **Kawaguchi, Toshio**
**2-22-2234 Seibu Danchi, 3910, Ohba**
**Fujisawa-shi, Kanagawa-ken (JP)**
Inventor: **Nakahara, Takeshi**
**103 Sakura Height 0-64-4 Shonan Life Town**
**1235 Endo Fujisawa-shi Kanagawa-ken (JP)**
Inventor: **Kunimoto, Shinichiro**
**203 Co-op Tsukasa 1723 Endo**
**Fujisawa-shi Kanagawa-ken (JP)**

(74) Representative: **Woods, Geoffrey Corlett et al**
**J.A. KEMP & CO. 14 South Square Gray's Inn**
**London WC1R 5EU (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an adhesive coating material for a hard tissue.

Various compounds are known as adhesives applicable peculiarly in various fields. Among these adhesives, especially severe requirements are imposed on dental adhesives used to hard tissues in humid condition. Since a dental adhesive is used in the oral cavity, a high adhesion strength in the humid condition is required. Furthermore, this dental adhesive should have an adhesive force to both of hard tissue and a dental resinous restorative material comprising a polymerizable monomer and at least one meber selected from an inorganic, organic and inorganic/organic composite fillers.

As dental adhesives, an ionomer cement comprising an aqueous solution of polyacrylic acid and inorganic oxide, and a cold-setting adhesive comprising a polymerizable monomer are widely used.

The ionomer cement has an adhesive force to the hard tissue, but no adhesive force to a dental resinous restorative material. And the ionomer cement is readily separated from the bonded portion since its water resistance is low.

The cold-setting adhesive comprising a polymeriable monomer hardly adheres to the hard tissue without pretreatment. Accordingly, it is necessary that the hard tissue should be treated with a high concentrated aqueous solution of phosphoric acid to prepare a mechanical retentive surface. This method, however, is defective in that even healthy hard tissue is damaged since phosphoric acid is used at a high concentration.

Special properties are required for adhesives according to application fields thereof, and a certain adhesive usable in a certain field can seldom be used industrially in other fields. Accordingly, special adhesives are used in respective application fields, and also in the field of the dental treatment, development of an adhesive satisfying the foregoing requirements is eagerly desired.

DE—A—2739282 discloses adhesive materials for hard substrates comprising a radical polymerisable monomer and a hardener system comprising a peroxide, an amine and a sulphinic acid salt.

GB—A—1495255 refers to surgical cements which are homopolymers or copolymers of a poly(carboxylic acid anhydride) and contain an ion-washable powder.

EP—A—0017937 describes hard tissue bonding materials comprising polymerisable (meth)acrylate esters or (meth)acrylamide derivatives and alkoxy containing titanium compounds or organic silicon compounds.

Under the above-mentioned background, we made research with a view to developing a satisfactory adhesive coating material for a hard tissue, and we have now completed the present invention.

It is therefore a primary object of the present invention to provide an adhesive for a hard tissue, which can be bonded to a hard tissue directly without a pretreatment with an aqueous solution of phosphoric acid.

Another object of the present invention is to provide an adhesive which can be bonded with a sufficient adhesive force to a tooth and a dental resinous restorative material even in the oral cavity in the humid condition and which has a high water resistance.

In accordance with the present invention, there is provided an adhesive coating material suitable for a hard tissue, which comprises,

(1) a polymer having an acid value of 30 to 700 and including a hydrophobic group and two carboxyl (—COOH) groups or one carboxylic anhydride

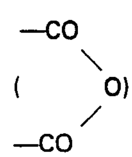

group bonded to the polymer, said carboxyl groups or the carbon atoms of the carboxylic anhydride group being bonded to adjacent carbon atoms;

(2) a polymerizable vinyl compound represented by the formula;

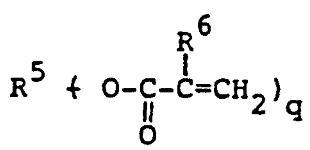

wherein $R^5$ stands for an organic group free of an ethylenic unsaturation, $R^6$ stands for a hydrogen atom or an alkyl group, and q is an integer of from 1 to 4;

(3) as a radical initiator, an organic peroxide and an amine;

(4) as a promotor, a sulfinic acid salt and/or carboxylic acid salt;

(5) optionally, an organic titante, and

(6) optionally, a stabiliser for the organic titanate.

In Application no. 83304729.3 (EP—A—0103420) from which the present application has been divided, we have described and claimed another adhesive coating material comprising a polymer (1) and an organic titanate.

One main component of the adhesive coating material of the present invention is a polymer including a hydrophobic group and two carboxyl groups or one carboxylic anhydride group bonded to the polymer, said carboxyl groups or carboxylic anhydride group being bonded to adjacent carbon atoms. The reason why the hydrophobic groups introduced into the polymer used in the present invention is that a sufficient water resistance is given to the adhesive coating material, a good compatibility or affinity with a resin to be bonded, for exmaple, a dental resinous restorative material, is imparted to the adhesive coating material and a sufficient adhesion strength is obtained even in the humid condition. Furthermore, in order to attain a sufficient adhesive strength to the hard tissue even in the humid condition, it is necessary that the two carboxyl groups or one carboxylic anhydride group should be bonded to adjacent carbon atoms in the polymer to be used in the present invention. Since the two carboxyl groups or one carboxylic anhydride group forms a bridge of a high strength with the organic titanate compound which may be used in the present invention, an especially high water resistance is given to the adhesive coating material of the present invention. Moreover, in the adhesive coating of the present invention, since the polymerizable vinyl monomer is polymerized in the state where the coating material is uniformly impregnated with the polymerizable vinyl compound, the adhesion strength of the adhesive coating material of the present invention is highly improved. Accordingly, the adhesive coating material of the present invention acts effectively as an adhesive between a hard tissue and a dental resinous restorative material such as a composite resin, especially in the humid condition.

It is also important that the polymer that is used in the present invention should have an acid value of 30 to 700, especially 40 to 600. In the instant specification, the acid value is defined as the number of mg of KOH necessary for neutralizing 1 g of the polymer. This acid value indicates the concentration of the carboxy group or carboxylic anhydride group in the polymer. If the acid value is smaller than 30, the adhesiveness to the hard tissue is reduced and the number of crosslinking points to the organic titanate compound is reduced, with the result that the toughness of the resulting coating is degraded. If the acid value is larger than 700, the coating formed from the polymer is excessively hydrophilic, and the water resistance is reduced. A polymer having an acid value within the above-mentioned range is especially suitable as a dental adhesive coating material.

The kind of the hydrophobic group bonded to the polymer is not particularly critical in the present invention, and any of known hydrophobic groups may be used. As preferred examples of the hydrophobic group, there can be mentioned (1) aryl groups such as phenyl and naphthyl groups, (2) alkyl groups such as methyl, ethyl, propyl and butyl groups, (3) alkoxy groups such as ethoxy, propoxy and butoxy groups, and (4) acryloxy groups such as an acetyloxy group. These hydrophobic groups (1) through (4) may be substituted with other substituents, such as halogen atoms, e.g., chlorine, bromine, iodine and fluorine, and alkyl, alkoxy and phenoxy groups.

In many cases, the hydrophobic group is ordinarily introduced into the polymer from a starting material used for the production of the polymer, as described hereinafter. The kind of the hydrophobic group-giving starting material is not particularly critical, and a known vinyl monomer is preferably used. More specifically, there are preferably used styrene, halogenostyrene, methylstyrene, halogenomethylstyrene and vinylnaphthalene as the monomer having the hydrophobic group (1), propylene and isobutene as the monomer having the hydrophobic group (2), ethylvinyl ether and n-butyl ether as the monomer having the hydrophobic group (3) and vinyl acetate as the monomer having the hydrophobic group (4).

A monomer for introduction of the hydrophobic group, which is especially preferred in the present invention, is represented by the following general formula:

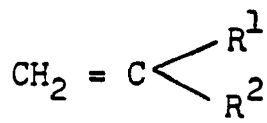

wherein $R^1$ stands for a hydrogen atom or an alkyl group, and $R^2$ stands for an aryl group, an alkyl group, an alkoxy group or an acyloxy group.

In the polymer used in the present invention, it is sufficient if the carboxyl groups or carboxylic anhydride group is finally bonded to adjacent carbon atoms, irrespectively of the preparation process. Ordinarily, a polymer having two carboxyl groups or one carboxylic anhydride group bonded thereto is preferably prepared by performing homopolymerization or copolymerization by using as the starting material a vinyl compound having two carboxyl groups or one carboxylic anhydride group bonded to adjacent carbon atoms, such as maleic acid, fumaric acid, itaconic acid, maleic anhydride or itaconic anhydride. Furthermore, there is preferably adopted a process in which a vinyl monomer having a carboxylic acid ester group or carboxylic anhydride group bonded to adjacent carbon atoms, such as maleic anhydride, maleic acid monoester, maleic acid diester, fumaric acid monoester or fumaric acid diester is used as one polymerizable component and copolymerized with other copolymerizable vinyl

monomer, and the carboxylic anhydride group or carboxylic acid ester group of the formed copolymer is hydrolyzed to convert all or a part of the carboxylic anhydride group or carboxylic acid ester group to a carboxyl group.

The kind of the polymer that is used in the present invention is not particularly critical, so far as it includes a hydrophobic group as mentioned above and two carboxyl groups or one carboxylic anhydride group and the two carboxyl groups or the carbon atoms of the carboxylic anhydride group are bonded to adjacent carbon atoms. In view of the availability and the handling easiness, it is industrially preferred that a polymer having an average molecular weight of 1,000 to 100,000, especially 2,000 to 50,000, be used. The process for the preparation of this polymer is not particularly critical. From the industrial viewpoint, however, there is preferably adopted a process in which a vinyl monomer having a hydrophobic group, such as mentioned above, is copolymerized with a vinyl monomer having a carboxyl group, a carboxylic acid ester group or a carboxylic anhydride group bonded to adjacent carbon atoms and, if necessary, the obtained polymer is then hydrolyzed.

A preferred polymer comprises (A) at least one kind of monomeric units represented by the following formula:

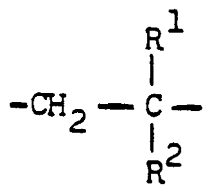

wherein $R^1$ stands for a hydrogen atom or an alkyl group, and $R^2$ stands for an aryl group, an alkyl group, an alkoxy group, an acyloxy group or an alkoxycarbonyl group,
and (B) at least one kind of monomeric units represented by the following formula:

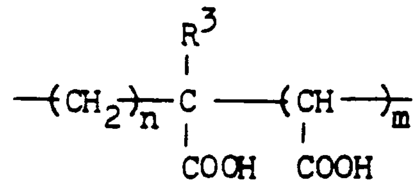

wherein $R^3$ stands for a hydrogen atom or a carboxymethyl group, n and m are numbers of zero or 1, with the proviso that when n is zero, mis 1 and $R^3$ is a hydrogen atom and when n is 1, m is zero and $R^3$ is a carboxymethyl group, and the two carboxyl groups may form a carboxylic acid anhydride group.

The above-mentioned copolymerization is not particularly critical. Ordinarily, however, there is preferably adopted a polymerization process in which a polymerization initiator described below is used. More specifically, there is preferably adopted a radical polymerization process using an organic peroxide such as benzoyl peroxide or lauroyl peroxide, an azo compound such as azobisisobutyronitrile, an organic metal compound such as tributyl boron or a redox type initiator.

In the above-mentioned copolymer, it is preferred that the vinyl monomer component having the hydrophobic group be contained in an amount of 40 to 90 mole %. If the content of the hydrophobic group-containing vinyl monomer is higher than 90 mole %, no satisfactory adhesive force to a hard tissue can be obtained. The reason s not sufficiently elucidated, but it is presumed that since the hydrophobic characteristics of the copolymer are increased, the affinity with the hard tissue will be reduced. If the hydrophobic group-containing vinyl monomer is contained in the copolymer in an amount smaller than 40 mole %, there is not a suitable process for imparting a carboxyl group or carboxylic anhydride group to this copolymer, and therefore, the production of the copolymer is difficult and the water resistance of an adhesive comprising this polymer is not sufficient. As is apparent from the foregoing description, the content of the hydrophobic group-containing vinyl monomer in the copolymer used in the present invention is very important.

The above-mentioned process in which the carboxylic acid ester group or carboxylic anhydride group is hydrolyzed is not particularly critical. Ordinarily, there is preferably adopted a process in which a copolymer comprising maleic anhydride, maleic acid diester, fumaric acid diester or itaconic acid diester is dissolved in an organic solvent, water and a small amount of an alkaline or acid component as a hydrolysis promotor are added to the solution and the reaction is carried out at room temperature or under heating.

A polymerizable vinyl compound is used as another component of the adhesive coating material of the present invention. The polymerizable vinyl compound is represented by the following formula:

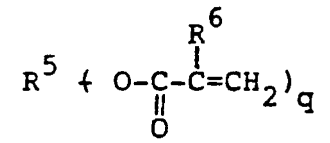

wherein $R^5$ stands for an inorganic group free of an ethylenic unsaturation, $R^6$ stands for a hydrogen atom or an alkyl group, and q is an integer of form 1 to 4.

For example, there can be used monovinyl compounds such as methyl acrylate, methyl methacrylate, 2-hydroxyethyl acrylate, 2-hydroxyethyl methacrylate, 2-hydroxypropyl acrylate, 2-hydroxypropyl methacrylate and glycidyl methacrylate, and polyvinyl compounds such as diacrylic acid esters and dimethacrylic acid esters of ethylene glycol, diethylene glycol, triethylene glycol, tetraethylene glycol, dipropylene glycol and butylene glycols, triacrylic acid esters and trimethacrylic acid esters of trimethylolpropane and trimethylolethane, tetraacrylic acid esters and tetramethacrylic acid esters of tetramethylolmethane, and bisphenol-A-diglycidyl methacrylate (hereinafter referred to as "bis-GMA"). These monovinyl compounds and polyvinyl compounds can be used singly or in the form of mixtures of two or more of them.

In view of the toughness, water resistance and adhesiveness of the formed coating, it is preferred that the polymerizable vinyl compound or at least one polymerizable vinyl compound when a plurality of polymerizable vinyl compounds are used be a vinyl compound having at least two ethylenic unsaturations, and in the present invention, it is especially preferred that one or more of polymerizable vinyl compounds be used so that the functional index (F.I.) defined by the following formula:

$$F.I. = \sum_{m=1}^{n} m.Mm$$

wherein m stands for the number of vinyl groups (ethylenic unsaturations) in the vinyl compound and Mm stands for a molar fraction of the vinyl compound containing m of vinyl groups, is 1.0 to 3.5, especially 1.1 to 3.3, particularly especially 1.3 to 3.0. If the F.I. value is smaller than 1.0, the toughness of the formed coating is degraded, and if the F.I. value is larger than 3.5, the formed coating is relatively brittle.

From the view point of the adaptability of the composite resin-filling operation conducted after formation of the coating, it is preferred that the polymerizable vinyl compound should contain a hydroxyl group in an amount of 50 to 800 millimoles, especially 70 to 600 millimoles, per 100 g of the vinyl compound. If the hydroxyl group concentration is too low and below the above range, the filled composite resin is not bonded to the formed coating and is readily separated, and if the hydroxyl group concentration is too high and exceeds the above range, the water resistance of the formed coating is insufficient.

Optionally, an organic titanate compound may be used in the adhesive coating material of the present invention. The kind of the organic titanate compound is not particularly critical, and any of known organic titanate compounds can be used. For example, there can be mentioned tetra-isopropyl titanate, tetra-n-butyl titanate, tetrakis(2-ethylhexyl) titanate, tetrastearyl titanate, di-iso-propoxy-bis(acetylacetone) titanate, di-n-butoxybis(triethanolamine) titanate, dihydroxy-bis(lactic acid) titanate, tetraoctylene glycol titanate, tri-n-butoxymonostearyl titanate, isopropyl-triiso-stearoyl titanate, isopropyltridency-benzenesulfonyl titanate, isopropyl-tris(dioctylpyrophosphate) titanate, tetra-iso-propyl-bis(dioctyl phosphite) titanate, tetraoctyl-bis(ditridecyl phosphite) titanate, tetra-(2,2-diallyloxymethyl-1-butyl)-bis(ditridecyl phosphite) titanate, bis(dioctyl pyrophosphate)oxyacetate titanate and bis(dioctyl pyrophosphate)ethylene titanate. These organic titanate compounds may be used singly or in the form of mixtures of two or more of them. Moreover, polymers of these titanates may be used.

In the present invention, an organic titanate represented by the following formula:

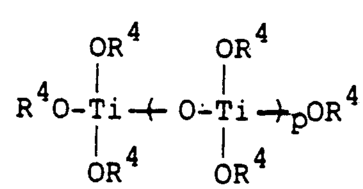

wherein $R^4$ stands for an alkyl group and p is a number of from 0 to 20, especially a tetraalkyl titanate, is preferably used as the orgnaic titanate compound.

The mixing ratio of the three main components of the adhesive coating material of the present invention, above-mentioned polymer (1), the polymerizable vinyl compound (2) and the optional organic titanate compound, is not particularly critical, so far as the intended adhesive effect is attained. However, it

is ordinarily preferred that the polymer be incorporated in an amount of 0.1 to 40% by weight based on the polymerizable vinyl compound. If the organic titanate compound is used, it is preferred that the amount used of the organic titanate compound be used in an amount of 0.02 to 2 moles per mole of the unit having bonded carboxyl group or carboxylic anhydride group in the polymer.

In the present invention, in order for the adhesive coating material to exert sufficient functions, it is necessary that a catalyst should be added. Any of known catalysts can be used, so far as it is capable of polymerizing the vinyl compond. Ordinarily a radical initiator, for example, a mixture of a peroxide and an amine, is preferably used. A peroxide customarily used as a curing agent can be used, and dibenzoyl peroxide (hereinafter referred to as "BPO") and dilauroyl peroxide are preferably used. As the amine, there are preferably used N,N'-dimethylaniline, N,N'-dimethyl-p-toluidine (hereinafter referred to as "DMPT"), N-methyl-N'-β-hydroxyethylaniline, N,N'-dimethyl-p-(β-hydroxyethyl)aniline and N,N'-di(β-hydroxyethyl)-p-toluidine (hereinafter referred to as "DEPT").

A sulfinic acid salt and/or a carboxylic acid salt is used as a promotor together with the radical initiator. Good results are ofter obtained. The kind of the sulfinic acid salt is not particularly critical, and known sulfinic acid salts may be used. However, it is ordinarily preferred that a salt of a sulfinic acid bonded to an alkyl or aryl group be used. As especially preferred examples, there can be mentioned alkali metal salts such as sodium and potassium salts, alkaline earth metal salts such as calcium and strontium salts, ammonium salts, trialkyl ammonium salts and N,N'-dimethyl-p-toluidine salts of benzene-sulfinic acid, p-toluene-sulfinic acid, β-naphthalene-sulfinic acid and styrene-sulfinic acid. Any of known metal salts of carboxylic acids can be used as another type of the promotor without any limitation. Furthermore, a monobasic carboxylic acid or a polybasic carboxylic acid such as a dicarboxylic acid or a tricarboxylic acid may be used. In case of a polybasic carboxylic acid, a poly-metal salt such as a di-metal salt is preferably used rather than a mono-metal salt. As specific examples of the carboxylic acid component of the promotor there can be mentioned aliphatic carboxylic acids such as formic acid, acetic acid, propionic acid, butyric acid, valeric acid, lauric acid, stearic acid, pyruvic acid, methoxyacetic acid and acetoacetic acid, aromatic carboxylic acids such as benzoic acid, phenylacetic acid and salicylic acid, hydroxycarboxylic acids such as glycolic acid and lactic acid, dicarboxylic acids such as oxalic acid, succinic acid, adipic acid, tartaric acid, maleic acid, fumaric acid and phthalic acid, and vinyl carboxylic acids such as acrylic acid, methacrylic acid and vinylacetic acid. As the carboxylic acid salt, there are preferably used alkali metal salts such as sodium and potassium salts, alkaline earth metal salts such as magnesium and calcium salts, iron salts, copper salts, zinc salts and silver salts of the above-mentioned carboxylic acids.

In the present invention, the amounts used of the radical initiator and promotor are not particularly critical, and they are appropriately determined according to the kinds of the polymerizable vinyl monomer, radical initiator and promotor and application conditions. Ordinarily, the radical initiator is used in an amount of 0.01 to 3% by weight based on the polymerizable vinyl compound, and the promotor is used in an amount of 40 to 600% by weight based on the radical initiator.

In the case where the organic titanate compound is used as one component of the adhesive coating material of the present invention, in order to use the adhesive coating material in a more stable state, it is preferred that a stabilizer for the organic titanate compound be used. Especially in the case where a solvent containing water is used when the adhesive coating material of the present invention is actually applied, it is often preferred that a stabilizer for the organic titanate compound be used. An appropriate stabilizer is selected and used according to the application mode of the adhesive coating material of the present invention. For example, (A) an o-alkoxybenzoic acid such as o-methoxybenzoic acid, o-ethoxybenzoic acid or o-propoxybenzoic acid, or a β-hydroxycarboxylic acid such as hydroacrylic acid, β-hydroxybutyric acid or β-hydroxyisovaleric acid; or (B) an α-hydroxycarboxylic acid such as lactic acid, α-hydroxy-n-butyric acid or mandelic acid, a β-hydroxyalkyl acrylate or methacrylate such as β-hydroxyethyl methacrylate, β-hydroxypropyl acrylate or glycerin dimethacrylate, a catechol derivative such as catechol, guaiacol or eugenol, a proline derivative such as proline, 4-methylene-proline or 4-methyl-proline, or a cyclic ester such as β-butyrolactone, γ-butyrolactone or β-caprolactone; may be used as the stabilizer for the organic titanate compound.

In order to prevent deterioration of the polymerizable vinyl compound, it is necessary that the organic peroxide and amine compound of the radical initiator should be contained in different vessels. Accordingly, in this embodiment, there sould be adopted a two-pack or three-pack type product system in which the ingredients are mixed at the time of application.

When an organic titanate is not used, a two-pack product may be employed. The above-mentioned polymer (1), the polymerizable vinyl compound (2) and the organic peroxide are contained in one vessel, and the amine compound is contained in another vessel, if necessary in the form of a solution in a solvent. At the time of application the contents of both the vessels are mixed together. The sulfinic acid salts and/or carboxylic acid salt used as the promotor are preferably contained in the vessel where the amine compound is contained.

A two-pack product may be employed when an organic titanate and a stabiliser of type (A) above are used. The polymer (1), polymerizable vinyl compound (2), organic titanate, stabilizer and organic peroxide are contained in one vessel and the amine compound ans sulfinic acid salt and/or carboxylic acid salt are contained in another vessel. These components are used in the form of solvent solutions, if necessary. At

the time of application, appropriate amounts of the contents of both the vessels are taken out and are mixed together.

A three-pack product may be employed when an organic titanate compound and a stabilizer of type (B) above are used. The organic titanate compound and the stabiliser are contained in still another vessel, not in the vessel containing the polymer (1), polymerizable vinyl compound (2) and organic peroxide or in the vessel containing the amine and the promoter.

The kind of the solvent which may be used is not particularly critical, and an appropriate solvent is selected among known solvents.

Ordinarily, there are preferably used alcohols such as ethyl alcohol and isopropyl alcohol, esters such as ethyl acetate, and dioxane and tetrahydrofuran. The amount added of the stabilizer for the organic titanate compound differs according to the kind of the stabilizer and it is not sweepingly determined. However, it is ordinarily preferred that the stabilizer be used in an amount of 0.1 to 4 moles, especially 0.5 to 2 moles, per mole of the organic titanate compond. The stabilizer may be mixed with the organic titanate compound in advance or it may be added to the organic titanate compound together with other ingredients.

As is apparent from the foregoing description, the adhesive coating material of the present invention has a very high adhesiveness to a hard tissue and a dental resinous restorative material, and this adhesiveness is excellent even in the humid condition. Accordingly, the adhesive coating material of the present invention can be used especially advantageously as a dental restorative material. By the term "dental restorative material" is meant a material which is used for remedy and restoration of a tooth and is coated on the surface of the tooth or the surface of the cavity formed on the tooth, and this is the most important use for the adhesive coating material of the present invention. As the dental restorative material, there can be mentioned, for example, a tooth adhesive, a protective lining material for the dental pulp and a border sealer for a tooth and a restorative material.

An embodiment where the adhesive coating material of the present invention is used as a dental restorative material will now be described.

In the conventional method of remedy and restoration of teeth, when a restorative composite resin is filled in a cavity of a tooth, an adhesive is used for bonding the restorative material to the hard tissue. However, since a conventional adhesive has no substantial adhesiveness to the hard tissue, it is necessary to treat the hard tissue in advance with an aqueous solution of phosphoric acid to prepare a mechanical retentive surface. This method, however, is defective in that since an aqueous solution of phosphoric acid having a high concentration is used, even the healthy hard tissue is damaged. Especially when the dentin is etched, attainment of any substantial adhesive force cannot be expected and it is anticipated that even the pulp will be influenced by the aqueous solution of phosphoric acid introduced through a ductless gland of the dentin. Furthermore, in the method described before, since a residual monomer is inevitably left, there is a risk of damage of the pulp by this monomer.

In contrast, when the adhesive coating material of the present invention is used, since the pretreatment with an aqueous solution of phosphoric acid is not necessary and the adhesive coating material can directly be bonded to the dentin, there is no risk of damage of the pulp by a residual monomer.

A lining material of the calcium hydroxide type or a cement has heretofore been used as the protecting lining material for the pulp. This lining material is used for protecting the dentin from the phosphoric acid etching conducted at the step of filing a restorative material such as a restorative composite resin. However, if the conventional lining material is used, increase of the thickness of the coating cannot be avoided, and the conventional lining material has no adhesive force to a filler. Accordingly, the conventional lining material can hardly be used for filling of a shallow cavity in a tooth. In contrast, when the adhesive coating material of the present invention is dissolved in an organic solvent and is used as the lining material, the dentin can be protected from the phosphoric acid etching even though the formed coating layer is very thin, and furthermore, the material exerts an excellent function of being bonded to a restorative material.

A zinc phosphate cement which is often used for bonding a metal to the hard tissue involves a risk of damage of the pulp because it contains a large amount of phosphoric acid. Therefore, the treatment with this cement inherently needs the use of a lining material for protection of the dentin. However, in case of the conventional lining material providing a thick coating layer, the compressing strength of the coating material is insufficient and the conventional lining material cannot be used for this purpose. When the adhesive coating material is used as a lining material of the above-mentioned type, since the coating layer is very thin, a high compressive strength is not necessary for the coating layer, and moreover, this thin coating layer does not allow permeation of phosphoric acid. Accordingly, the adhesive coating material exerts ideal effects.

As the third function of the adhesive coating material, there can be mentioned the improvement of a border sealing property.

As the known substance expected to exert this function, there can be mentioned, for example, a solution of a resin such as copalite (tradename) in an organic solvent which is used for filling of amalgam. Indeed, this material provides a thin coating layer, but the material has no adhesive force to the dentin or amalgam, and no substantial border sealing effect can be attained. In contrast, if the adhesive coating material of the present invention is used as a border sealer, a high border sealing effect can be attained. In

view of the fact that the adhesive coating material of the present invention can be bonded to the dentin but cannot be bonded to amalgam, it is considered that the above function is due to properties other than the adhesiveness, for example, the close adhesion and hydrophobic property.

When the adhesive coating material of the present invention is used for the filling operations other than the amalgam filling, for example, the restorative composite resin filling, the cement filling and the rubber capping, the border sealing effect can be improved.

The adhesive coating material of the present invention can be applied to uses other than the abovementioned uses. For example, when it is intended to remove the material filled in the cavity of a tooth or to restore a wedge-shaped defect on the neck of the tooth, if the adhesive coating material is coated, it can effectively act as an insulating material to external stimulant.

The functions of the adhesive coating material of the present invention as the dental adhesive, the protective lining material for the pulp and the border sealer have independently been described. Since the adhesive coating material of the present invention has these functions in combination, if the coating material of the present invention is applied to the treatment of one case, all of these functions can be exerted at one time. According to the conventional technique, a plurality of materials should be used in combination for the treatment of one case, and hence, the operation becomes complicated, and the functions of the respective materials are degraded because of the combined application of the materials. If this fact is taken into consideration, it will readily be understood that the adhesive coating material of the present invention is very valuable as a dental restorative material.

When the adhesive coating material of the present invention is used as a dental restorative material, it is important that the polymer used as one component should have a carboxyl group or carboxylic anhydride group in addition to the hydrophobic group, and because of the presence of the carboxyl group or carboxylic acid anhydride group, the function as the dental restorative material is further improved. Namely, the water resistance is improved so that the above function can sufficiently be exerted even under the severe condition in the oral cavity, that is, 100% relative humidity. When the adhesive coating material is used as an adhesive for bonding a composite restorative resin to the dentin, since the carboxyl group or carboxylic anhydride group has an affinity with the dentin and the hydrophobic group has an affinity with the composite restorative resin, the adhesive force is highly improved over adhesive forces attainable by conventional adhesives.

The present invention will now be described in detail with reference to the following Examples that by no means limit the scope of the invention.

Production Example 1

A 500 ml-capacity glass separable flask was charged with 200 ml of cyclohexane, and 5.2 g of styrene, 4.9 g of maleic anhydride and 0.05 g of benzoyl peroxide (hereinafter referred to as "BPO") were added and the mixture was sufficiently stirred.

Then, the pressure in the vessel was reduced and the atmosphere was replaced with nitrogen, and the mixture was heated at 80°C with stirring for 4 hours to effect polymerization. The reaction mixture was cooled to room temperature and the formed precipitate was recovered by filtration. The obtained solid was sufficiently washed with 300 ml of benzene and dried to obtain 8.7 g of a white polymer. When the composition of the formed copolymer was determined by the elementary analysis, it was found that the copolymer comprised 48.4 mole % of styrene and 51.6 mole % of maleic anhydride.

Then, the product was dissolved in 80 ml of dioxane, and the solution was charged into a 500 ml-capacity flask and 100 ml of an aqueous solution of potassium hydroxide having a concentration of 5% by weight was added to the solution with sufficient stirring. Reaction was carried out at room temperature for 10 hours. Then, concentrated hydrochloric acid was added to the reaction mixture to effect neutralization, and an excessive amount of hydrochloric acid was further added to obtain a white solid precipitate. The solid was recovered by filtration and it was washed with water repeatedly until the solid became neutral, and the solid was dried to obtain 8.0 g of a copolymer. When the product was subjected to the infrared absorption spectrum analysis, it was found that characteristic absorptions attributed to the carbonyl group of maleic anhydride at 1850 $cm^{-1}$ and 1775 $cm^{-1}$ completely disappeared and a characteristic absorption attributed to the carbonyl group of maleic acid newly appeared at 1720 $cm^{-1}$. Thus, it was confirmed that the hydrolysis reaction was advanced substantially quantitatively. Namely, it was confirmed that the obtained white solid was a copolymer comprising 48.4 mole % of styrene and 51.6 mole % of maleic acid. Incidentally, the acid value of the polymer was 370.

Production Example 2

A solution of 10 g of a commercially available styrene-maleic anhydride copolymer having a molecular weight of 50,000 (supplied by Monsanto Co.) in 200 ml of dioxane was charged in a 500 ml-capacity flask, and 10 g of distilled water was added to the solution with sufficient stirring and the mixture was heated at 100°C for 4 hours with stirring. The solution was cooled to room temperature and thrown into 2 liters of distilled water to precipitate a white cotton-like polymer. When the polymer was washed with water and dried, 9.8 g of a white solid was obtained. From the results of the elementary analysis and infrared absorption spectrum analysis, it was confirmed that a styrene-maleic acid copolymer was obtained. Incidentally, the acid value of the polymer was 367.

Production Examples 3 and 4

In the same manner as described in Production Example 1, two commercially available styrene-maleic anhydride copolymer (supplied by Arco Chemical Co.) differing in the composition as shown in Table 1 were hydrolyzed. From the results of the elementary analysis of the starting copolymers and the results of the infrared absorption spectrum analysis of the hydrolyzed copolymers, it was confirmed that styrene-maleic acid copolymers having compositions shown in Table 1 were obtained. The molecular weights of the copolymers were 1,700 and 1,900, and the acid values of the copolymers were 251 and 184.

TABLE 1

| Production Example No. | Tradename of Commercially Available Maleic Anhydride-Styrene Copolymer | Composition of Styrene-Maleic Acid Copolymer | |
|---|---|---|---|
| | | styrene (mole %) | maleic acid (mole %) |
| 3 | SMA 2000 | 66.7 | 33.3 |
| 4 | SMA 3000 | 75.1 | 24.9 |

Production Example 5

A pressure glass vessel having an inner capacity of 300 ml was charged with 50 ml of benzene containing 35 g of maleic anhydride and 90 mg of azobisisobutyronitrile (hereinafter referred to as "AIBN"), and the inner atmosphere was replaced by nitrogen under reduced pressure while cooling in a dry ice-methanol bath. Then, 12 g of propylene was introduced into the reaction vessel by distillation through a liquefaction meter, and the mixture was stirred at 60°C for 36 hours to effect copolymerization. After the termination of the polymerization, the content was thrown into a large amount of anhydrous ether to precipitate the formed copolymer. The polymer was sufficiently washed by decantation and promptly dried in a reduced pressure dryer. The yield was 60%. From the results of the elementary analysis, it was found that the copolymer comprised 55.6 mole % of maleic anhydride and 44.4 mole % of propylene.

Then, the product was hydrolyzed in the same manner as described in Production Example 1 to obtain 24.2 g of a propylene-maleic acid copolymer. From the results of the infrared absorption spectrum analysis, it was confirmed that the maleic anhydride group in the starting copolymer was converted to maleic acid substantially quantitatively. Incidentally, the acid value of the polymer was 508.

Production Example 6

A pressure glass vessel having an inner capacity of 300 ml was charged with 50 ml of benzene containing 35.7 g of maleic anhydride and 90 mg of AIBN, and 12.5 g of isobutene was introduced into the reaction vessel by distillation through a liquefaction meter and copolymerization was carried out at 60°C for 15 minutes. After the termination of the polymerization, the content was thrown into a large amount of anhydrous ether to precipitate the formed copolymer, and the supernatant was discarded by decantation and the residue was sufficiently washed with anhydrous ether and then dried under reduced pressure. The yield was 43.3%. From the results of the elementary analysis, it was found that the product was a copolymer comprising 47.1 mole % of isobutene and 52.9 mole % of maleic anhydride.

In the same manner as described in Production Example 1, the product was hydrolyzed to obtain 20.5 g of an isobutene-maleic acid copolymer. From the results of the infrared absorption spectrum analysis of the copolymer, it was confirmed that the maleic anhydride group in the starting copolymer was converted to maleic acid substantially quantitatively. Incidentally, the acid value of this copolymer was 470.

Production Example 7

A 500 ml-capacity glass separable flask was charged with 200 ml of benzene, and 5.3 g of n-butylvinyl ether, 4.9 g of maleic anhydride and 0.05 g of AIBN were added and the mixture was sufficiently stirred.

Then, the pressure in the vessel was reduced and the inner atmosphere was replaced by nitrogen, and heat polymerization was carried out at 60°C for 6 hours and the formed precipitate was recovered by filtration. From the results of the elementary analysis, it was found that the formed copolymer comprised 49.8 mole % of n-butyl vinyl ether and 50.2 mole % of maleic anhydride. Then, the product was dissolved in 200 ml of dioxane and the solution was charged in a 500 ml-capacity flask, and 10 g of distilled water was added under sufficient stirring and the mixture was heated and stirred at 60°C for 2 hours. The obtained polymer solution was solidified by dry ice-methanol and then freeze-dried to obtain 10.1 g of a white solid. From the results of the infrared absorption spectrum analysis of the product, it was confirmed that the majority of the maleic anhydride group in the starting copolymer was converted to a maleic acid group. Incidentally, the acid value of the polymer was 375.

Production Example 8

A commercially available n-octadecylvinyl ether-maleic anhydride copolymer (supplied by Polysciences, Inc.) was hydrolyzed in the same manner as described in Production Example 2. From the results of the elementary analysis of the starting copolymer and the results of the infrared absorption spectrum analysis of the hydrolyzed copolymer, it was confirmed that the product was an n-octadecylvinyl ether-maleic acid copolymer having an acid value of 196.

Production Example 9

In 200 g of dioxane were dissolved 30 g of itaconic acid and 20 g of styrene, and BPO was added to the solution in an amount of 0.1% based on the monomers and polymerization was carried out at 10°C for 5 hours. The reaction mixture was thrown in 1 liter of hexane, and the precipitate was recovered by filtration, dried and washed with distilled water to remove unreacted itaconic acid. The yield was 4.2%. From the results of the of the elementary analysis, it was found that the product was a copolymer comprising 49.0 mole % of itaconic acid and 51.0 mole % of styrene. The acid value of the polymer was 340.

Production Example 10

Styrene was copolymerized with diethyl fumarate at 60°C for 20 hours by using AIBN as the initiator. From the results of the elementary analysis of the formed polymer, it was found that the formed polymer was a copolymer comprising 56.5 mole % of styrene and 43.5 mole % of diethyl fumarate. Then, 0.5 g of the polymer was charged in an Erlenmeyer flask having an inner capacity of 100 ml, and 30 ml of concentrated sulfuric acid was added and the mixture was allowed to stand still. In 2 days, the polymer was completely dissolved and a yellow solution was obtained. When then solution was poured into a large amount of ice water, a styrene-fumaric acid copolymer was precipitated. The precipitate was recovered by filtration, washed sufficiently with water repeatedly and dried to obtain 0.45 g of a solid. The acid value of the polymer was 93.

Production Example 11

A commercially available vinyl acetate-maleic anhydride copolymer (supplied by Polysciences, Inc.) was hydrolyzed in the same manner as described in Production Example 7. From the results of the elementary analysis of the starting copolymer and the results of the infrared absorption spectrum analysis of the hydrolyzed copolymer, it was found that the product was a vinyl acetate-maleic acid copolymer. The acid value of the polymer was 399.

Production Example 12

p-Chlorostyrene was copolymerized with maleic anhydride by using BPO as the initiator under the same conditions as in Production Example 1. From the results of the elementary analysis of the obtained copolymer, it was found that the obtained copolymer comprised 47.9 mole % of p-chlorostyrene and 52.1 mole % of maleic anhydride. The product was hydrolyzed in the same manner as described in Production Example 7. From the results of the elementary analysis of the formed polymer and the results of the infrared absorption spectrum analysis of the hydrolyzed polymer, it was confirmed that the product was a vinyl acetate-maleic acid copolymer. The acid value of this polymer was 318.

Production Example 13

p-Chloromethylstyrene was copolymerized with maleic anhydride by the using BPO as the initiator under the same conditions as adopted in Production Example 1. From the results of the elementary analysis of the obtained copolymer, it was found that the formed copolymer comprised 48.9 mole % of p-chloromethylstyrene and 51.1 mole % of maleic anhydride. The product was hydrolyzed in the same manner as described in Production Example 7. From the results of the elementary analysis of the formed copolymer and the results of the infrared absorption spectrum analysis of the hydrolyzed copolymer, it was confirmed that the product was a p-chloromethylstyrenemaleic acid copolymer. The acid value of this polymer was 301.

Example 1

Liquid (I) shown in Table 2 was prepared by using a commercially available styrene/maleic anhydride copolymer (SMA 1000 supplied by Arco Chemical), and liquid (II) was prepared by adding the catyalyst and promotor shown in Table 2 to 100 parts by weight of ethanol. The adhesive strength of an adhesive coating material formed from these liquids (I) and (II) was determined according to the following procedures.

Pastes (A) and (B) were prepared according to the following recipes.

Paste (A):

| | |
|---|---|
| Bis-GMA | 11.0 parts by weight |
| Triethylene glycol dimethacrylate (hereinafter referred to as "TEGDMA") | 10.5 parts by weight |
| DMPT | 0.5 part by weight |
| Silane-treated quartz powder (having particle size smaller than 80 microns) | 78.0 parts by weight |

Paste (B):

| | |
|---|---|
| Bis-GMA | 11.0 parts by weight |
| TEDMA | 10.5 parts by weight |
| BPO | 1.0 part by weight |
| Silane-treated quartz powder (having particle size smaller than 80 microns) | 78.0 parts by weight |

The lip side surface of a freshly extracted bovine tooth was polished by emery paper (#320) to expose a smooth dentin, and nitrogen gas was blown to dry the surface. Then, a plate-like wax having a through hole having a diameter of 4 mm was attached to the dried surface by using a double-coated tape. Then, the adhesive liquids (I) and (II) were mixed at a ratio of 1:1 and the mixture was coated on the dentin surface surrounded by the plate-like wax, and nitrogen was blown to sweep off the excessive adhesive. Then, the pastes (A) and (B) were mixed at a ratio of 1:1 and the mixture was filled onto the adhesive coating. After standing for 1 hour, the plate-like wax was removed, and the treated tooth was dipped in water maintained at 37°C for 24 hours and the tensile strength was measured at a pulling speed of 10 mm/min by using Tensilon supplied by Toyo-Baldwin. The obtained results are shown in Table 2.

Incidentally, the trifunctional monomer and polyfunctional monomer referred to in Table 2 and subsequent Tables are the following monomers.

Trifunctional Monomer:

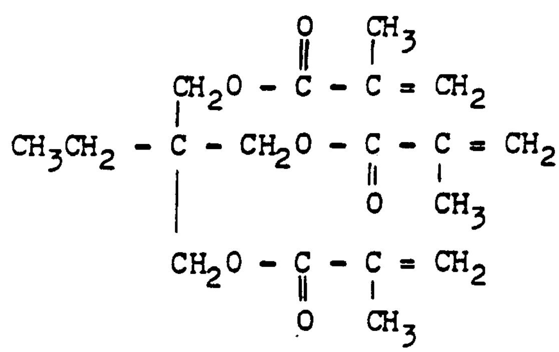

Polyfunctional Monomer:

The polyfunctional monomer is a mixture of the following monomers (i), (ii) and (iii):

(i) 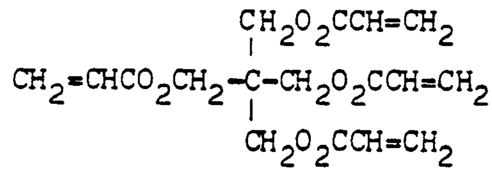 (50 % by weight)

(ii) 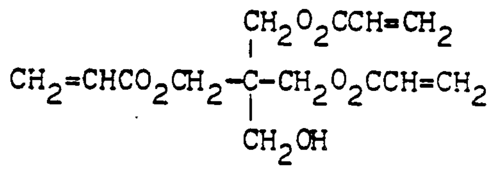 (45 % by weight)

(iii) 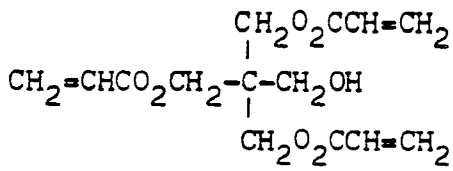 (5 % by weight)

TABLE 2

| Run No. | Polymer (parts by weight) | Liquid (I) | Liquid (II) | | | Adhesive Strength (× 981 Kg · $cm^{-1}$. $s^{-2}$) to Dentin |
|---|---|---|---|---|---|---|
| | | Polymerizable Vinyl Compound (parts by weight) (F.I.) | Peroxide Catalyst (parts by weight) | Amine Catalyst (parts by weight) | Promotor (parts by weight) | |
| 1 | SMA 100 (10) | 2-hydroxyethyl methacrylate (90 (1.0) | BPO (1) | DEPT (1.5) | sodium p-toluene-sulfinate (3.0) | 20.5 |
| 2 | ditto | triethylene glycol dimeth-acrylate (90) (2.0) | ditto | ditto | ditto | 37.1 |
| 3 | ditto | 2-hydroxyethyl methacrylate (30) (1.0), triethylene glycol dimethacrylate (60) (1.5) | ditto | ditto | ditto | 39.2 |
| 4 | ditto | 2-hydroxyethyl methacrylate (25) (1.0), triethylene glycol dimethacrylate (55) (1.5), bis-GMA (35) (1.6) | ditto | ditto | ditto | 34.2 |
| 5 | ditto | 2-hydroxyethyl methacrylate (34) (1.0), triethylene glycol dimethacrylate (76) (1.5), bis-GMA (14) (1.6) | ditto | ditto | ditto | 35.5 |
| 6 | ditto | triethylene glycol dimeth-acrylate (15) (1.5), bis-GMA (21) (1.6), trifunctional monomer (54) (2.6) | ditto | ditto | ditto | 31.1 |
| 7 | ditto | triethylene glycol dimeth-acrylate (15) (1.5), bis-GMA (21) (1.6), polyfunctional monomer (54) (2.9) | ditto | ditto | ditto | 34.2 |

Example 2

The adhesive strength was measured in the same manner as described in Example 1 except that liquid (I) was prepared by using the polymer obtained in one of Production Examples 1 through 13, as shown in Table 3, instead of the polymer used in Example 1. The obtained results are shown in Table 3.

TABLE 3

| Run No. | Liquid (I) | | | Liquid (II) | | |
|---|---|---|---|---|---|---|
| | Polymer (parts by weight) | Polymerizable Vinyl Monomer (parts by weight) (F.I.) | Peroxide Catalyst (parts by weight) | Amine Catalyst parts by weight) | Promotor (parts by weight) | Adhesive Strength (× 981 kg. cm$^1$. s$^{-2}$) to Dentin |
| 1 | copolymer of Production Example 1 (10) | 2-hydroxyethyl methacrylate (30) (1.0), triethylene glycol dimethacrylate (60) (1.5) | BPO (1) | DEPT (1.5) | sodium p-toluene sulfinate (3.0) | 38.2 |
| 2 | copolymer of Production Example 2 (5) | ditto | ditto | ditto | ditto | 31.6 |
| 3 | copolymer of Production Example 3 (15) | ditto | ditto | ditto | ditto | 37.1 |
| 4 | copolymer of Production Example 4 (12) | ditto | ditto | ditto | ditto | 35.5 |
| 5 | copolymer of Production Example 5 (10) | ditto | ditto | ditto | ditto | 24.0 |
| 6 | copolymer of Production Example 6 (10) | ditto | ditto | ditto | ditto | 25.4 |
| 7 | copolymer of Production Example 7 (5) | ditto | ditto | ditto | ditto | 28.9 |

TABLE 3 (continued)

| Run No. | Liquid (I) | | Liquid (II) | | | Adhesive Strength (× 981 kg. $cm^1$. $s^{-2}$) to Dentin |
|---|---|---|---|---|---|---|
| | Polymer (parts by weight) | Polymerizable Vinyl Monomer (parts by weight) (F.I.) | Peroxide Catalyst (parts by weight) | Amine Catalyst parts by weight) | Promotor (parts by weight) | |
| 8 | copolymer of Production Example 8 (8) | 2-hydroxyethyl methacrylate (30) (1.0), triethylene glycol dimethacrylate (60) (1.5) | BPO (1) | DEPT (1.5) | sodium p-toluene sulfinate (3.0) | 19.8 |
| 9 | copolymer of Production Example 9 (10) | ditto | ditto | ditto | ditto | 39.1 |
| 10 | copolymer of Production Example 10 (10) | ditto | ditto | ditto | ditto | 30.5 |
| 11 | copolymer of Production Example 11 (10) | ditto | ditto | ditto | ditto | 28.8 |
| 12 | copolymer of Production Example 12 (10) | ditto | ditto | ditto | ditto | 37.1 |
| 13 | copolymer of Production Example 13 (10) | ditto | ditto | ditto | ditto | 36.5 |

Example 3

The procedures of Example 2 (Run No. 1 in Table 3) were repeated in the same manner except that the promotor used in Example 2 (Run No. 1 in Table 3) was changed as indicated in Table 4. The obtained results are shown in Table 4. Incidentally, Runs Nos. 4, 6 and 7, water was added for dissolution of the promotor. The amounts added of water in Runs Nos. 4, 6 and 7 were 5, 50 and 100 parts by weight, respectively, per 100 parts by weight of the ethanol solution.

TABLE 4

| Run No. | Liquid (I) | | | Liquid (II) | | Adhesive Strength (× 981 kg. $cm^{-1}$. $s^{-2}$) | |
|---|---|---|---|---|---|---|---|
| | Polymer (parts by weight) | Polymerizable Vinyl Monomer (parts by weight) | Peroxide Catalyst (parts by weight) | Amine Catalyst parts by weight) | Promotor parts by weight) | to dentin | to enamel |
| 1 | SMA 1000 (10) | 2-hydroxyethyl methacrylate (30), triethylene glycol dimethacrylate (60) | BPO (1) | DEPT (1.5) | sodium p-toluene sulfinate (3.0) | 39.2 | 107.5 |
| 2 | ditto | ditto | BPO (0.5) | DEPT (0.75) | sodium p-toluene-sulfinate (1.5) | 34.3 | 124.3 |
| 3 | ditto | ditto | BPO (1) | DEPT (1.5) | potassium methacrylate (1.2) | 33.7 | |
| 4 | ditto | ditto | ditto | ditto | potassium methacrylate (3.0) | 38.3 | 113.1 |
| 5 | ditto | ditto | ditto | ditto | sodium p-toluene-sulfinate (1.0), potassium methacrylate (1.0) | 29.8 | |
| 6 | ditto | ditto | ditto | DEPT (2.3) | sodium laurate (4.5) | 15.7 | |
| 7 | ditto | ditto | ditto | DEPT (3.0) | copper acetate (6.0) | 21.4 | |

Example 4

Liquids (I), (II) and (III) shown in Table 5 were prepared by using a commercially available styrene/maleic anhydride copolymer (SMA 1000), a polymerizable vinyl compound shown in Table 5, tetra-n-butyl titanate (TBT) as the organic titanate compound, β-hydroxyethyl methacrylate as the stabilizer for the organic titanate compound and sodium p-toluenesulfinate as the promotor. Incidentally, each of the amounts of the organic titanate comound stabilizer and promotor used for the liquids (II) and (III) is expressed as parts by weight per 100 parts by weight of ethanol. The adhesive strength was measured by using liquids (I), (II) and (III) shown in Table 5 in the same manner as described in Example 1. The obtained results are shown in Table 5.

TABLE 5

| Run No. | Polymer (parts by weight) | Liquid (I): Polymerizable Vinyl Compound (parts by weight) (F.I.) | Peroxide Catalyst (parts by weight) | Liquid (II): Organic Titanate Compound (parts by weight) | Liquid (II): Stabilizer (parts by weight) |
|---|---|---|---|---|---|
| 1 | SMA 1000 (10) | 2-hydroxyethyl methacrylate (90) (1.0) | BPO (1) | TBT (2) | 2-hydroxyethyl methacrylate (1) |
| 2 | ditto | 2-hydroxyethyl methacrylate (30) (1.0), triethylene glycol dimethacrylate (60) (1.5) | ditto | ditto | ditto |
| 3 | ditto | 2-hydroxyethyl methacrylate (25 (1.0), triethylene glycol dimethacrylate (55) (1.5), bis-GMA (35 (1.6) | ditto | ditto | ditto |
| 4 | ditto | triethylene glycol dimethacrylate (15 (1.5), bis-GMA (21 (1.6), trifunctional monomer (54) (2.6) | ditto | ditto | ditto |
| 5 | ditto | triethylene glycol dimethacrylate (15) (1.5), bis-GMA (21) (1.6), polyfunctional monomer (54) (2.9) | ditto | ditto | ditto |

TABLE 5 (Continued)

| Run No. | Liquid (III): Amine Catalyst (parts by weight) | Liquid (III): Promotor (parts by weight) | Adhesive Strength (× 981 kg $cm^{-1}.S^{-2}$) to Dentin |
|---|---|---|---|
| 1 | DEPT (1.5) | sodium p-toluenesulfinate (3.0) | 30.1 |
| 2 | ditto | ditto | 43.2 |
| 3 | ditto | ditto | 39.0 |
| 4 | ditto | ditto | 35.0 |
| 5 | ditto | ditto | 38.0 |

Example 5

Liquids (I) and (II) shown in Table 6 were prepared by using the styrene-maleic acid copolymer prepared in Production Example 1, a polymerizable vinyl compound shown in Table 6, tetra-n-butyl titanate (TBT) as the organic titanate compound, o-ethoxybenzoic acid as the stabilizer for the organic titanate compound and sodium p-toluene-sulfinate as the promotor. Incidentally, in the liquids (I) and (II), each of the amounts of the polymer, organic titanate compound, stabilizer and promotor is expressed as parts by weight per 100 parts by weight of ethanol. Equal amounts of the liquids (I) and (II) were mixed, and the adhesive strength was measured in the same manner as described in Example 1. The obtained results are shown in Table 6.

TABLE 6

| Run No. | Polymer (parts by weight) | Liquid (I) | | | Stabilizer (parts by weight | Liquid (II) | | Adhesive Strength (× 981 kg $cm^{-1}.S^{-2}$) to Dentin |
|---|---|---|---|---|---|---|---|---|
| | | Polymerizable Vinyl Compound (parts by weight) | Peroxide Catalyst (parts by weight) | Organic Titanate Compound (parts by weight) | | Amine Catalyst parts by weight | Promotor (parts by weight | |
| 1 | copolymer of Production Example 1 (5.3) | 2-hydroxyethyl methacrylate (100) | BPO (1) | tetra-n-butyl titanate (1.1) | o-ethoxybenzoic acid (0.6) | DEPT (1.5) | sodium p-toluenesulfinate (3.0) | 30.3 |
| 2 | ditto | 2-hydroxyethyl methacrylate (50), glycerin dimethacrylate (50) | ditto | ditto | ditto | ditto | ditto | 41.3 |

Example 6

Liquids (I), (II) and (III) shown in Table 7 were prepared by using one of the copolymers obtained in Production Examples 1 through 13, 2-hydroxyethyl methacrylate/triethylene glycol dimethacrylate as the polymerizable vinyl compound, tetra-n-butyl titanate as the organic titanate compound, eugenol as the titanate stabilizer and sodium p-toluene-sulfinate as the promotor. Incidentally, in the liquid (I), each of the amounts of the ingredients is expressed as parts by weight per 20 parts by weight of ethanol, and in the liquids (II) and (III), each of the amounts of the ingredients is expressed as parts by weight per 100 parts by weight of ethanol. The liquids (I), (II) and (III) were mixed, and the adhesive strength was measured in the same manner as described in Example 1. The obtained results are shown in Table 7.

TABLE 7

| Run No. | Liquid (I) | | | Liquid (II) | |
|---|---|---|---|---|---|
| | Polymer (parts by weight) | Polymerizable Vinyl Compound (parts by weight) | Peroxide Catalyst (parts by weight) | Organic Titanate Compound (parts by weight) | Stabilizer (parts by weight) |
| 1 | copolymer of Production Example 1 (10) | 2-hydroxyethyl methacrylate (30), triethylene glycol dimethacrylate (60) | BPO (1) | tetra-n-butyl titanate (2) | eugenol (1) |
| 2 | copolymer of Production Example 2 (5) | ditto | ditto | tetra-n-butyl titanate (1) | eugenol (0.5) |
| 3 | copolymer of Production Example 3 (15) | ditto | ditto | tetra-n-butyl titanate (3) | eugenol (1.5) |
| 4 | copolymer of Production Example 4 (12) | ditto | ditto | tetra-n-butyl titanate (2) | eugenol (1) |
| 5 | copolymer of Production Example 5 (10) | ditto | ditto | ditto | ditto |
| 6 | copolymer of Production Example 6 (10) | ditto | ditto | ditto | ditto |
| 7 | copolymer of Production Example 7 (5) | ditto | ditto | ditto | ditto |

TABLE 7 (continued)

| Run No. | Liquid (III): Amine Catalyst (parts by weight) | Liquid (III): Promotor (parts by weight) | Adhesive Strength ($\times$ 981 kg $cm^{-1}.S^{-2}$) to Dentin |
|---|---|---|---|
| 1 | DEPT (1.5) | sodium p-toluene-sulfinate (3.0) | 41.5 |
| 2 | ditto | ditto | 34.6 |
| 3 | ditto | ditto | 40.2 |
| 4 | ditto | ditto | 38.7 |
| 5 | ditto | ditto | 28.0 |
| 6 | ditto | ditto | 29.0 |
| 7 | ditto | ditto | 32.5 |

TABLE 7 (Continued)

| | Liquid (I) | | | Liquid (II) | |
|---|---|---|---|---|---|
| Run No. | Polymer (parts by weight) | Polymerizable Vinyl Compound (parts by weight) | Peroxide Catalyst (parts by weight) | Organic Titanate Compound (parts by weight) | Stabilizer (parts by weight) |
| 8 | copolymer of Production Example 8 (8) | 2-hydroxy ethyl methacrylate (30), triethylene glycol dimethacrylate (60) | BPO (1) | tetra-n-butyl titanate (2) | eugenol (1) |
| 9 | copolymer of Production Example 9 (10) | ditto | ditto | ditto | ditto |
| 10 | copolymer of Production Example 10 (10) | ditto | ditto | ditto | ditto |
| 11 | copolymer of Production Example 11 (10) | ditto | ditto | ditto | ditto |
| 12 | copolymer of Production Example 12 (10) | ditto | ditto | ditto | ditto |
| 13 | copolymer of Production Example 13 (10) | ditto | ditto | ditto | ditto |

TABLE 7 (continued)

| Run No. | Liquid (III) Amine Catalyst (parts by weight) | Promotor (parts by weight) | Adhesive Strength ($\times$ 981 kg $cm^{-1}.S^{-2}$) to Dentin |
|---|---|---|---|
| 8 | DEPT (1.5) | sodium-toluene-sulfinate (3.0) | 24.1 |
| 9 | ditto | ditto | 42.5 |
| 10 | ditto | ditto | 33.9 |
| 11 | ditto | ditto | 32.5 |
| 12 | ditto | ditto | 40.8 |
| 13 | ditto | ditto | 40.1 |

Example 7

Liquids (I), (II) and (III) shown in Table 8 were prepared by using the copolymer of Production Example 1, 2-hydroxyethyl methacrylate/triethylene glycol dimethacrylate as the polymerizable vinyl compound, an organic titanate compound shown in Table 8, o-ethoxybenzoic acid as the stabilizer and sodium p-toluenesulfinate as the Promotor. The liquids (I), (II) and (III) were mixed at a time, and the adhesive strength was measured at a time, and the adhesive strength was measured in the same manner as described in Example 1. The obtained results are shown in Table 8. Incidentally, in the liquid (I), each of the amounts of the ingredients is expressed as parts by weight per 10 parts by weight of ethanol, and in the liquids (II) and (III), each of the amounts of the ingredients is expressed as parts by weight per 100 parts by weight of ethanol.

TABLE 8

| Run No. | Liquid (I) | | | Liquid (II) | |
|---|---|---|---|---|---|
| | Polymer (parts by weight) | Polymerizable Vinyl Compound (parts by weight) | Peroxide Catalyst (parts by weight) | Organic Titanate Compound parts by weight) | Stabilizer (parts by weight) |
| 1 | copolymer of Production Example 1 (10) | 2-hydroxyethyl methacrylate (30), triethylene glycol dimethacrylate (60) | BPO (1) | tetra-n-butyl titanate (2) | o-ethoxybenzoic acid (1) |
| 2 | ditto | ditto | ditto | tetraisopropyl titanate (2) | ditto |
| 3 | ditto | ditto | ditto | tetrakis(2-ethylhexyl) titanate (2) | ditto |
| 4 | ditto | ditto | ditto | dimer of tetra-n-butyl titanate (2) | ditto |
| 5 | ditto | ditto | ditto | dimer of tetra-n-butyl titanate (3) | ditto |
| 6 | ditto | ditto | ditto | tetramer of tetra-n-butyl titanate (2) | ditto |

TABLE 8 (Continued)

| Run No. | Liquid (III) Amine Catalyst (parts by weight) | Liquid (III) Promotor (parts by weight) | Adhesive Strength (× 981 $kg \cdot cm^{-1} \cdot s^{-2}$) to Dentin |
|---|---|---|---|
| 1 | DEPT (1.5) | sodium p-toluene-sulfinate (3.0) | 41.3 |
| 2 | ditto | ditto | 41.5 |
| 3 | ditto | ditto | 40.8 |
| 4 | ditto | ditto | 40.5 |
| 5 | ditto | ditto | 39.9 |
| 6 | ditto | ditto | 40.2 |

Example 8

Liquids (I), (II) and (III) shown in Table 9 were prepared by using the styrene/maleic acid copolymer of Production Example 1, 2-hydroxyethyl methacrylate/triethylene glycol dimethacrylate as the polymerizable vinyl compound, tetra-n-butyl titanate (TBT) as the organic titanate compound, 2-hydroxyethyl methacrylate as the stabilizer for the organic titanate compound and a promotor shown in Table 9. Incidentally, in the liquid (I), each of the amounts of the ingredients is expressed as parts by weight per 10 parts by weight of ethanol, and in the liquids (II) and (III), each of the amounts of the ingredients is expressed as parts by weight per 100 parts by weight of ethanol. Equal amounts of the liquids (I), (II) and (III) were mixed at a time, and the adhesive strength was measured in the same manner as described in Example 1. The obtained results are shown in Table 9.

TABLE 9

| Run No. | Liquid (I) | | Liquid (II) | | |
|---|---|---|---|---|---|
| | Polymer (parts by weight) | Polymerizable Vinyl Compound (parts by weight) | Peroxide Catalyst (parts by weight) | Organic Titanate Compound (parts by weight) | Stabilizer (parts by weight) |
| 1 | copolymer of Production Example 1 (10) | 2-hydroxyethl methacrylate (30), triethylene glycol dimethacrylate (60) | BPO (1) | tetra-n-butyl titanate (2) | 2-hydroxyethyl methacrylate (1) |
| 2 | ditto | ditto | ditto | ditto | ditto |
| 3 | ditto | ditto | ditto | ditto | ditto |

TABLE 9 (Continued)

| Run No. | Liquid (III) Amine Catalyst (parts by weight) | Liquid (III) Promotor (parts by weight) | Adhesive Strength (× 981 $kg \cdot cm^{-1}s^{-2}$) to Dentin |
|---|---|---|---|
| 1 | DEPT (1) | sodium p-toluene-sulfinate (3.0) | 41.5 |
| 2 | ditto | potassium methacrylate (1.2) | 40.5 |
| 3 | ditto | sodium p-toluenesulfinate (1.0), potassium methacrylate (1.0) | 32.7 |

Example 9

By using liquids (I) and (II) for the first treatment, shown in Table 10, an untreated dentin of a bovine tooth was subjected to the coating treatment in the same manner as described in Example 1. Then, the coated surface was further coated with a liquid mixture of liquids (I') and (II') for the second treatment, shown in Table 10. Namely, liquid (I') comprising 10 parts by weight of a commercially available styrene/maleic anhydride copolymer (SMA 1000), 90 parts by weight of 2-hydroxyethyl methacrylate and 1 part by weight of BPO was mixed with liquid (II') comprising 1.5 parts by weight of DEPT, 3.0 parts by weight of sodium p-toluene-sulfinate and 100 parts by weight of ethanol at a ratio of 1:1, and the resulting liquid mixture was coated on the coated surface of the tooth. The adhesive test was carried out in the same manner as described in Example 1. The obtained results are shown in Table 10.

TABLE 10

First Treatment

| | Liquid (I) | Liquid (II) | |
|---|---|---|---|
| Run No. | Polymer (parts by weight) | Organic Titanate (parts by weight) | Stabilizer (parts by weight) |
| 1 | copolymer of Production Example 1 (10) | tetra-n-butyl titanate (2) | lactic acid (1) |
| 2 | ditto | ditto | 2-hydroxyethyl methacrylate (1) |
| 3 | ditto | ditto | eugenol (1) |
| 4 | ditto | ditto | proline (1) |
| 5 | ditto | ditto | o-ethoxybenzoic acid (1) |
| 6 | ditto | ditto | β-hydroxybutyric acid |

TABLE 10 (Continued)

| | Second Treatment | | | | | |
|---|---|---|---|---|---|---|
| | Liquid (I') | | | Liquid (II') | | |
| Run No. | Polymer (parts by weight) | Polymerisable Vinyl Compound (parts by weight) | Peroxide Catalyst (parts by weight) | Amine Catalyst (parts by weight) | Promotor (parts by weight) | Adhesive Strength (× 981 $Kg \cdot cm^{-1} \cdot s^{-2}$) to Dentin |
| 1 | SMA-1000 (10) | 2-hydroxyethyl methacrylate (30), triethylene glycol dimethacrylate (60) | BPO (1) | DEPT (1.5) | sodium p-toluenesulfinate (3.0) | 45.6 |
| 2 | ditto | ditto | ditto | ditto | ditto | 46.0 |
| 3 | ditto | ditto | ditto | ditto | ditto | 45.0 |
| 4 | ditto | ditto | ditto | ditto | ditto | 38.5 |
| 5 | ditto | ditto | ditto | ditto | ditto | 48.9 |
| 6 | ditto | ditto | ditto | ditto | ditto | 44.1 |

**Claims**

1. An adhesive coating material suitable for a hard tissue, which comprises,

(1) a polymer having an acid value of 30 to 700 and including a hydrophobic group and two carboxyl (—COOH) groups or one carboxylic anhydride

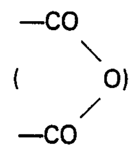

group bonded to the polymer, said carboxyl groups or the carbon atoms of the carboxylic anhydride group being bonded to adjacent carbon atoms;

(2) a polymerizable vinyl compound represented by the formula;

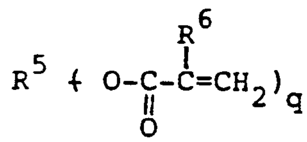

wherein $R^5$ stands for an organic group free of an ethylenic unsaturation, $R^6$ stands for a hydrogen atom or an alkyl group, and q is an integer of from 1 to 4;

(3) as a radical initiator, an organic peroxide and an amine;

(4) as a promotor, a sulfonic acid salt and/or carboxylic acid salt;

(5) optionally, and organic titanate and

(6) optionally, a stabiliser for the organic titanate (5).

2. An adhesive coating material according to claim 1, wherein the hydrophobic group is present in the polymer molecule in an amount of 0.7 to 9.0 moles per mole of the two carboxyl groups or one carboxylic anhydride group.

3. An adhesive coating material according to claim 1 or 2, wherein the average molecular weight of the polymer is 1,000 to 100,000.

4. An adhesive coating material according to any one of the preceding claims, wherein the hydrophobic group is at least one such group selected from aryl groups, alkyl groups, alkoxy groups and acyloxy groups.

5. An adhesive coating material according to any one of the preceding claims, wherein the polymer is a copolymer of a vinyl monomer having a hydrophobic group and a vinyl monomer having two carboxyl groups or one carboxylic anhydride group bonded to adjacent carbon atoms.

6. An adhesive coating material according to claim 5, wherein the vinyl monomer having a hydrophobic group is a monomer represented by the following general formula:

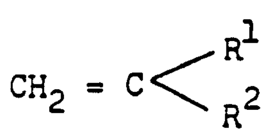

wherein $R^1$ stands for a hydrogen atom or an alkyl group and $R^2$ stands for an aryl group, an alkyl group, an alkoxy group or an acyloxy group.

7. An adhesive coating material according to claim 5 or 6, wherein the vinyl monomer having two carboxyl groups or one carboxylic anhydride group bonded to adjacent carbon atoms is selected from maleic acid, fumaric acid, itaconic acid, maleic anhydride and itaconic anhydride.

8. An adhesive coating material according to any one of claims 1 to 4, wherein the polymer comprises (A) at least one kind of monomeric unit represented by the following formula:

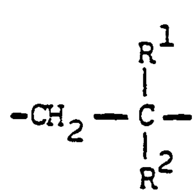

wherein $R^1$ stands for a hydrogen atom or an alkyl group, and $R^2$ stands for an aryl group, an alkyl group, an

alkoxy group, an acyloxy group or an alkoxycarbonyl group, and (B) at least one kind of monomeric unit represented by the following formula:

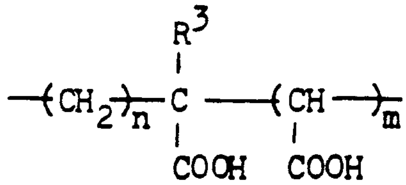

wherein $R^3$ stands for a hydrogen atom or a carboxymethyl group, n and m are each zero or 1 with the proviso that when n is zero, m is 1 and $R^3$ is a hydrogen atom and when n is 1, m is zero and $R^3$ is a carboxymethyl group, and the two carboxyl groups may form a carboxylic acid anhydride group.

9. An adhesive coating material according to any one of the preceding claims, wherein the polymer is present in an amount of 0.1 to 40% by weight based on the polymerizable vinyl compound.

10. An adhesive coating material according to any one of the preceding claims, wherein the polymerizable vinyl compound or at least one polymerizable vinyl compound when a plurality of polymerizable vinyl compounds are used is a vinyl compound having at least two ethylenic unsaturations.

11. An adhesive coating material according to any one of the preceding claims, wherein one or more polymerisable vinyl compound is used such that the functional index (F.I.) defined by the following formula:

$$F.I. = \sum_{m=1}^{n} m.Mm$$

wherein m stands for the number of vinyl groups (ethylenic unsaturations) in the vinyl compound and Mm stands for a molar fraction of the vinyl compound containing m of vinyl groups,

is from 1.1 to 3.3.

12. An adhesive coating material according to any one of the preceding claims which comprises 0.1 to 40% by weight of the polymer (1) based on the polymerizable vinyl compound (2); 0.02 to 2 moles of the organic titanate (5) per mole of the two carboxyl groups or one carboxylic anhydride group of the polymer (1); 0.01 to 3% by weight of the radical initiator (3) based on the polymerizable vinyl compound (2); and 40 to 600% by weight of the promotor (4) based on the radical initiator (3).

13. An adhesive coating material according to any one of claims 1 to 12 containing an organic titanate (5).

14. An adhesive coating material according to any one of claims 1 to 12 containing an organic titanate (5) and containing no stabiliser (6) for the organic titanate (5).

15. An adhesive coating material according to any one of claims 1 to 12 containing an organic titanate (5) and containing a stabiliser (6) for the organic titanate (5).

16. An adhesive coating material according to claim 15, wherein the stabiliser (6) is an o-alkoxybenzoic acid or β-hydroxylcarboxylic acid.

17. An adhesive coating material according to claim 15, wherein the stabiliser (6) is at least one stabiliser selected from α-hydroxycarboxylic acids, β-hydroxyalkyl acrylates, β-hydroxalkyl methacrylates, catechol derivatives, proline derivatives and cyclic ethers.

18. An adhesive coating material according to any one of claims 14 to 17 wherein the organic titanate (5) is a titanate represented by the following formula:

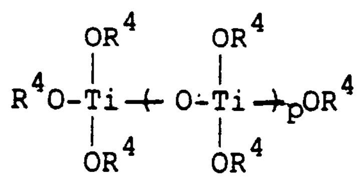

wherein $R^4$ stands for an alkyl group and p is 0 or an integer of from 1 to 20.

19. An adhesive coating material according to claims 14 to 17 wherein the organic titanate (5) is a tetraalkyl titanate.

20. A two-pack product for use to prepare an adhesive coating material, which product comprises a polymer (1) as defined in any one of claims 1 to 8, a vinyl compound (2) as defined in any one of claims 1, 10 and 11 and an organic peroxide component of a radical initiator (3) in a first vessel; and an amine

component of the radical initiator (3) and as a promotor (4), a sulfinic acid salt and/or a carboxylic acid salt in a second vessel.

21. A two-pack product according to claim 20, wherein the first vessel further contains an organic titanate (5) as defined in any one of claims 1, 18 and 19 and a stabilizer (6) as defined in claim 16.

22. A three-pack product for use to prepare an adhesive coating material which product comprises a polymer (1) as defined in any one of claims 1 to 8, a polymerizable vinyl compound (2) as defined in any one of claims 1, 10 and 11 and an organic peroxide component of a radical initiator (3) in a first vessel; an organic titanate (5) as defined in any one of claims 1, 18 and 19 and a stabiliser (6) as defined in claim 17 in a second vessel; and an amine component of the radical initiator (3) and, as a promotor (4), a sulfinic acid salt and/or a carboxylic acid salt in a third vessel.

23. Use of an adhesive coating material as claimed in any one of claims 1 to 19 or prepared from a product as claimed in any one of claims 20 to 22 a a dental restorative material.

**Patentansprüche**

1. Haftendes Überzugsmittel für harte Gewebe, umfassend (1) ein Polymer mit einem Säurewert vom 30 bis 700, und das eine hydrophobe Gruppe und zwei Carboxyl (—COOH)-Gruppen oder eine Carbonsäureanhydrid

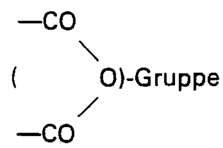

an das Polymer gebunden enthält, wobei die Carboxylgruppen oder die Kohlenstoffatome der Carbonsäureanhydridgruppe an benachbahrte Kohlenstoffatome gebunden sind;

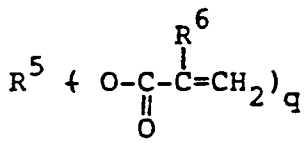

worin $R^1$ eine organische Gruppe bedeutet, die frei ist von einer ethylenischen Ungesättigtheit, $R^6$ eine Wasserstoffatom oder eine Alkylgruppe bedeutet, und q eine ganze Zahl von 1 bis 4 ist;

(3) als radikalischen Initiator ein organisches Peroxid und ein Amin;

(4) als Promotor ein Sulfinsäuresalz und/oder ein Carbonsäuresalz;

(5) gegebenenfalls ein organisches Titanat und

(6) gegebenenfalls einen Stabilisator für das organische Titanat (5).

2. Haftendes Überzugsmittel nach Anspruch 1, dadurch gekennzeichnet, daß die hydrophobe Gruppe im Polymermolekül in einer Menge von 0.7 bis 9.0 Mol pro Mol der zwei Carboxylgruppen oder einen Carbonsäureanhydridgruppe vorhanden ist.

3. Haftendes Überzugsmittel nach Anspruch 1 oder 2, dadurch gekennzeichnet, daß das durschnittliche Molekulargewicht des Polymers 1000 bis 100000 ist.

4. Haftendes Überzugsmittel nach einem der vorhergehenden Ansprüche, dadurch gekennzeichnet, daß die hydrophobe Gruppe mindestens eine solche Gruppe ist, ausgewählt aus Arylgruppen, Alkylgruppen, Alkoxygruppen und Acyloxygruppen.

5. Haftendes Überzugsmittel nach einem der vorhergehenden Ansprüche, dadurch gekennzeichnet, daß das Polymer ein Copolymer eines Vinylmonomers mit einer hydrophoben Gruppe und eines Vinylmonomers mit zwei Carboxylgruppen oder einer Carbonsäureanhydridgruppe, die an zwei benachbarte Kohlenstoffatome gebunden sind, ist.

6. Haftendes Überzugsmittel nach Anspruch 5, dadurch gekennzeichnet, daß das Vinylmonomer mit einer hydrophoben Gruppe ein durch die folgende allgemeine Formel dargestelltes Monomer ist

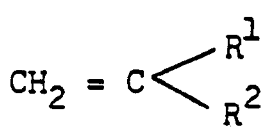

worin $R^1$ ein Wasserstoffatom oder eine Alkylgruppe bedeutet, und $R^2$ eine Arylgruppe, eine Alkylgruppe, eine Alkoxygruppe oder eine Acyloxygruppe ist.

7. Haftendes Überzugsmittel nach Anspruch 5 oder 6, dadurch gekennzeichnet, daß das Vinylmonomer

mit zwei Carboxylgruppen oder einer Carbonsäureanhydridgruppe, die an benachbarte Kohlenstoffatomen gebunden sind, ausgewählt ist aus Maleinsäure, Fumarsäure, Itaconsäure, Maleinsäureanhydrid und Itaconsäureanhydrid.

8. Haftendes Überzugsmittel nach einem der Ansprüche 1 bis 4, dadurch gekennzeichnet, daß das Polymer umfaßt (A) mindestens eine Art einer Monomereinheit der folgenden Formel:

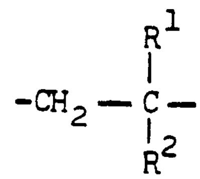

worin $R^1$ ein Wasserstoffatom oder eine Alkylgruppe bedeutet, und $R^2$ eine Arylgruppe, eine Alkylgruppe, eine Alkoxygruppe, eine Acyloxygruppe oder eine Alkoxycarbonylgruppe bedeutet, und (B) mindestens eine Art einer Monomereinheit der folgenden Formel:

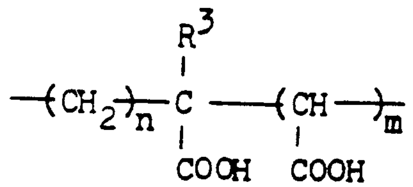

worin $R^3$ ein Wasserstoffatom oder eine Carboxymethylgruppe ist, n und m jedes null odr 1 sind, mit der Maßgabe, daß, wenn n null ist, m 1 ist und $R^3$ ein Wasserstoffatom ist, und wenn n 1 ist, m null und $R^3$ eine Carboxymethylgruppe ist, und die zwei Carboxylgruppen eine Carbonsäureanhydridgruppe bilden können.

9. Haftendes Überzugsmittel nach einem der vorhergehenden Ansprüche, dadurch gekennzeichnet, daß das Polymer in einer Menge von 0.1 bis 40 Gew. %, bezogen auf die polymerisierbare Vinylverbindung, vorhanden ist.

10. Haftendes Überzugsmittel nach einem der vorhergehenden Ansprüche, dadurch gekennzeichnet, daß die polymerisierbare Vinylverbindung oder zumindest eine polymerisierbare Vinylverbindung, wenn eine Vielzahl von polymerisierbaren Vinylverbindungen verwendet wird, eine Vinylverbindung mit mindestens zwei ethylenischen Ungesättigtheiten ist.

11. Haftendes Überzugsmittel nach einem der vorhergehenden Ansprüche, dadurch gekennzeichnet, daß eine oder mehrere polymerisierbare Vinylverbindungen verwendet werden, sodaß der durch die folgende Formel definierte funktionelle Index (F.I.):

$$F.I. = \sum_{m=1}^{n} m.Mm$$

worin m die Zahl der Vinylgruppen (ethylenische Ungesättigtheiten) in der Vinylverbindung ist und Mm den Molenbruch der Vinylverbindung, die m Vinylgruppen enthält, ist, 1.1 bis 3.3 beträgt.

12. Haftendes Überzugsmittel nach einem der vorhergehenden Ansprüche, dadurch gekennzeichnet, daß es umfaßt: 0.1 bis 40 Gew. % des Polymers (1), bezogen auf die polymerisierbare Vinylverbindung (2); 0.02 bis 2 Mol des organischen Titanats (5) pro Mol der zwei Carboxylgruppen oder einen Carbonsäureanhydridgruppe des Polymers (1); 0.01 bis 3 Gew. % des radikalischen Initiators (3), bezogen auf die polymierisierbare Vinylverbindung (2); und 40 bis 600 Gew. % des Promotors (4), bezogen auf den radikalischen Initiator (3).

13. Haftendes Überzugsmittel nach einem der Ansprüche 1 bis 12, dadurch gekennzeichnet, daß es kein organisches Titanat (5) enthält.

14. Haftendes Überzugsmittel nach einem der Ansprüche 1 bis 12, dadurch gekennzeichnet, daß es ein organisches Titanat (5) enthält und keinen Stabilisator (6) für das organische Titanat (5) enthält.

15. Haftendes Überzugsmittel nach einem der Ansprüche 1 bis 12, dadurch gekennzeichnet, daß es ein organisches Titanat (5 enthält und einen Stabilisator (6) für das organische Titanat (5) enthält.

16. Haftendes Überzugsmittel nach Anspruch 15, dadurch gekennzeichnet, daß der Stabilisator (6) eine o-Alkoxybenzolsäure oder β-Hydroxycarbonsäure ist.

17. Haftendes Überzugsmittel nach Anspruch 15, dadurch gekennzeichnet, daß der Stabilisator (6) mindestens ein Stabilisator ist ausgewählt aus α-Hydroxycarbonsäuren, β-Hydroxyalkylacrylaten, β-Hydroxyalkylmethacrylaten, Catecholderivaten, Prolinderivaten und cyclischen Ethern.

18. Haftendes Überzugsmittel nach einem der Ansprüche 14 bis 17, dadurch gekennzeichnet, daß das organische Titanat (5) ein durch folgende Formel dargestelltes Titanat ist:

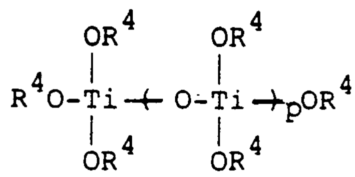

worin $R^4$ eine Alkylgruppe bedeutet und p 0 oder eine ganze Zahl von 1 bis 20 ist.

19. Haftendes Überzugsmittel nach den Ansprüchen 14 bis 17, dadurch gekennzeichnet, daß das organische Titanat (5) ein Tetraalkyltitanat ist.

20. Zweikomponentenprodukt zur Verwendung zur Herstellung eines haftenden Überzugsmittels, dadurch gekennzeichnet, daß das Produkt umfaßt: ein wie in einem der Ansprüche 1 bis 8 definiertes Polymer (1), eine wie in einem der Ansprüche 1, 10 und 11 definierte Vinylverbindung (2), und eine organische Peroxidkomponente eines radikalischen Initiators (3) in einem ersten Behälter; und eine Aminkomponente des radikalischen Initiators (3) und als Promotor (4) ein Sulfinsäuresalz und/oder ein Carbonsäuresalz in einem zweiten Behälter.

21. Zweikomponentenprodukt nach Anspruch 20, dadurch gekennzeichnet, daß der erste Behälter ferner ein wie in einem de Ansprüche 1, 18 und 19 definiertes organisches Titanat (5) und einen wie in Anspruch 16 definierten Stabilisator (5) enthält.

22. Dreikomponentenprodukt zur Verwendung zur Herstellung eines haftenden Überzugsmittels, dadurch gekennzeichnet, daß das Produkt umfaßt: ein wie in einem der Ansprüche 1 bis 8 definiertes Polymer (1), eine wie in einem der Ansprüche 1, 10 und 11 definierte polymerisierbare Vinylverbindung (2), und eine organische Peroxidkomponente eines radikalischen Initiators (3) in einem ersten Behälter; ein wie in einem der Ansprüche 1, 18 und 19 definiertes organisches Titanat (5) und einen wie ein Anspruch 17 definierten Stabilisator (6) in einem zweiten Behälter; und eine Aminkomponente des radikalischen Initiators (3) und als Promotor (4) ein Sulfinsäuresalz und/oder ein Carbonsäuresalz in einem dritten Behälter.

23. Verwendung eines haftenden Überzugsmittels nach einem der Ansprüche 1 bis 19 oder hergestellt aus einem Produkt nach einem der Ansprüche 20 bis 22 als dentales Restaurierungsmaterial.

**Revendications**

1. Matériau de revêtement adhésif convenant pour un tissu dur, ce matériau comprenant:

(1) un polymère ayant un indice d'acide 30 à 700 et contenant un groupe hydrophobe et deux groupes carboxyle (—COOH) ou un groupe anhydride carboxylique

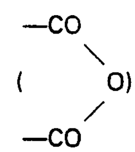

liés au polymère, les groupes carboxyle ou les atomes de carbone du groupe anhydride carboxylique étant liés à des atomes de carbone adjacents;

(2) un composé vinylique polymérisable représenté par la formule suivante:

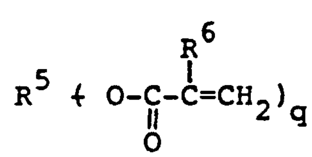

dans laquelle $R^5$ est un groupe organique dépourvu de double liaisons éthyléniques, $R^6$ est un atome d'hydrogène ou un groupe alcoyle et q est un nombre entier égal à 1, 2, 3 ou 4;

(3) un peroxyde organique et une amine en tant qu'initiateur de radicaux;

(4) un sel d'acide sulfinique et/ou un sel d'acide carboxylique en tant que promoteur;

(5) facultativement, un titanate organique; et

(6) facultativement, un stabilisant pour le titanate organique (5).

2. Matériau de revêtement adhésif selon la revendication 1, dans lequel le groupe hydrophobe est présent dans la molécule de polymère en une quantité de 0,7 à 9,0 moles par mole des deux groupes carboxyle ou du groupe anhydride carboxylique.

3. Matériau de revêtement adhésif selon la revendication 1 ou la revendication 2, dans lequel le poids moléculaire moyen du polymère est 1000 à 100 000.

4. Matériau de revêtement adhésif selon l'une quelconque des revendications précédentes, dans lequel le groupe hydrophobe est au moins un tel groupe choisi parmi les groupes aryle, les groupes alcoyle, les groupes alcoxy et les groupes acyloxy.

5. Matériau de revêtement adhésif selon l'une quelconque des revendications précédentes, dans lequel le polymère est un copolymère d'un monomère vinylique ayant un groupe hydrophobe et d'un monomère vinylique ayant deux groupes carboxyle ou un groupe anhydride carboxylique liés à des atomes de carbone adjacents.

6. Matériau de revêtement adhésif selon la revendication 5, dans lequel le monomère vinylique ayant un groupe hydrophobe est un monomère représenté par la formule générale suivante:

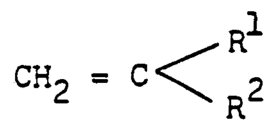

dans laquelle $R^1$ est un atome d'hydrogène ou un groupe alcoyle et $R^2$ est un groupe aryle, un groupe alcoyle, un groupe alcoxy ou un groupe acyloxy.

7. Matériau de revêtement adhésif selon la revendication 5 ou la revendication 6, dans lequel le monomère vinylique ayant deux groupes carboxyle ou un groupe anhydride carboxylique liés à des atomes de carbone adjacents est choisi parmi l'acide maléique, l'acide fumarique, l'acide itaconique, l'anhydride maléique et l'anhydride itaconique.

8. Matériau de revêtement adhésif selon l'une quelconque des revendications 1 à 4, dans lequel le polymère contient (A) un moins un type d'unité monomère représentée par la formule suivante:

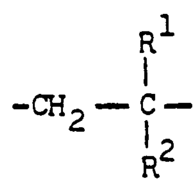

dans laquelle $R^1$ est un atome d'hydrogène ou un groupe alcoyle et $R^2$ est un groupe aryle, un groupe alcoyle, un groupe alcoxy, un groupe acyloxy ou un groupe alcoxycarbonyle, et (B) au moins un type d'unité monomère représentée par la formule suivante:

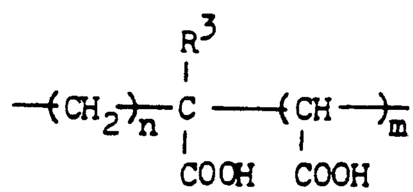

dans laqulle $R^3$ est un atome d'hydrogène ou un groupe carboxyméthyle, n et m sont chacun égaux à 0 ou à 1, sous réserve que, lorsque n est égal à 0, m est égal à 1 et $R^3$ est un atome d'hydrogène et, quand n est égal à 1, m est égal à 0 et $R^3$ est un groupe carboxyméthyle, et les deux groupes carboxyle peuvent former un groupe anhydride d'acide carboxylique.

9. Matériau de revêtement adhésif selon l'une quelconque des revendications précédentes, dans lequel le polymère est présent en une quantité de 0 à 40% en poids sur la base du composé vinylique polymérisable.

10. Matériau de revêtement adhésif selon l'une quelconque des revendications précédentes, dans lequel le composé vinylique polymérisable ou au moins un composé vinylique polymérisable, lorsqu'on utilise une multiplicité de composés vinylique polymérisables, est un composés vinylique ayant au moins deux doubles liaisons éthyléniques.

11. Matériau de revêtement adhésif selon l'une quelconque des revendications précédentes, dans lequel un ou plusieurs composés vinyliques polymérisables sont utilisés de façon que l'indice fonctionnel (F.I.) défini par la formule suivante:

$$F.I. = \sum_{m=1}^{n} m.Mm$$

dans laquelle m est le nombre de groupes vinyliques (doubles liaisons éthyléniques) dans le composé vinylique et Mm est une fraction molaire du composé vinylique contenant m groupes vinyliques, est compris entre 1,1 et 3,3.

12. Matériau de revêtement adhésif selon l'une quelconque des revendications précédentes, qui contient 0,1 à 40% en poids du polymère (1) sur la base du composé vinylique polymérisable (2); 0,02 à 2 moles du titanate organique (5) par mole des deux groupes carboxyle ou du groupe anhydride carboxylique du polymère (1); 0,01 à 3% en poids de l'initiateur de radicaux (3) sur la base du composé vinylique polymérisable (2); et 40 à 600% en poids du promoteur (4) sur la base de l'initiateur de radicaux (3).

13. Matériau de revêtement adhésif selon l'une quelconque des revendications 1 à 12, ne contenant pas de titanate organique (5).

14. Matériau de revêtement adhésif selon l'une quelconque des revendications 1 à 12, contenant un titanate organique (5) et ne contenant pas de stabilisant (6) pour le titanate organique (5).

15. Matériau de revêtement adhésif selon l'une quelconque des revendications 1 à 12, contenant un titanate organique (5) et contenant un stabilisant (6) pour le titanate organique (5).

16. Matériau de revêtement adhésif selon la revendication 15, dans lequele stabilisant (6) est un acide o-alcoxy benzoïque ou un acide β-hydroxycarboxylique.

17. Matériau de revêtement adhésif selon la revendication 15, dans lequel le stabilisant (6) est au moins un stabilisant choisi parmi les acides α-hydroxycarboxyliques, des acrylates de β-hydroxyalcoyle, les méthacrylates de β-hydroxyalcoyle, des dérivés de catéchol, des dérivés de proline et des éthers cycliques.

18. Matériau de revêtement adhésif selon l'une quelconque des revendications 14 à 17, dans lequel le titanate organique (5) est un titanate représenté par la formule suivante:

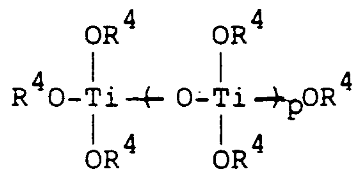

dans laquelle $R^4$ est un groupe alcoyle et p est égal à 0 ou est égal à un nombre entier de 1 à 20.

19. Matériau de revêtement adhésif selon la revendication 14 à 17, dans lequelle titanate organique (5) est un tétratitanate d'alcoyle.

20. Produit en deux emballages utilisé pour préparer un matériau de revêtement adhésif, lequel produit contient un polymère (1) tel que défini dans l'une quelconque des revendications 1 à 8, un composé vinylique (2) comme défini dans l'une quelconque des revedications 1, 10 et 11 et un peroxyde organique d'un initiateur de radicaux (3) dans un premier récipient; et une amine de l'initiateur de radicaux (3) et, en tant que promoteur (4), un sel d'acide sulfinique et/ou un sel d'acide carboxylique dans un deuxième récipient.

21. Produit en deux emballage selon la revendication 20, dans lequel le premier récipient contient en outre un titanate organique (5) tel que défini dans l'une quelconque des revendications 1, 18 et 19, et un stabilisant (6) tel que défini dans la revendication 16.

22. Produit en trois emballages utilisé pour préparér un matériau de revêtement adhésif, lequel produit contient un polymère (1) tel que defini dans l'une quelconque des revendications 1 à 8, un composé vinylique polymérisable (2) tel que défini dans l'une quelconque des revendications 1, 10 et 11, et un peroxyde organique d'un initiateur de radicaux (3) dans un premier récipient; un titanate organique (5) tel que défini dans l'une quelconque des revendications 1, 18 et 19, et un stabilisant (6) tel que défini dans la revendication 17 dans un deuxième récipient; et une amine de l'initiateur de radicaux (3) et, en tant que promoteur (4), un sel d'acide sulfinique et/ou un sel d'acide carboxylique dans un troisième récipient.

23. Utilisation d'un matériau de revêtement adhésif selon l'une des revendications 1 à 19 ou préparé à partir d'un produit tel que revendiqué dans l'une quelconque des revendications 20 à 22, comme matériau de reconstitution dentaire.